# EUROPEAN PATENT APPLICATION

(11) **EP 4 361 172 A2**
(43) Date of publication of application: **01.05.2024**
(21) Application number: 23195429.8
(22) Date of filing: 05.09.2023
(51) Int. Cl.: C07K 14/435, A61K 38/00, A61P 29/00

(54) **EXPRESSION VECTOR FOR RECOMBINANT U-CONOTOXIN TIIIA OR TIIIALAMUT EXPRESSION IN E.COLI**

(30) Priority: 27.10.2022 EP 22204012
(71) Applicant: Science4Beauty Sp. z o.o., 03-735 Warsaw (PL)
(72) Inventor: Mazurkiewicz Pisarek, Anna, 02 991 Warsaw (PL); Mikiewicz, Diana, 03 337 Warsaw (PL); Mazurkiewicz, Alina, 02 797 Warsaw (PL); Ciach, Tomasz, 02 765 Warsaw (PL)
(74) Representative: Kondrat, Mariusz

(57) **Abstract**

Subject matter of the invention is a construct for an expression vector for recombinant µ-conotoxin TIIIA or TIIIAlaMut expression, characterized in that it contains either the nucleotide sequence of µ-conotoxin TIIIA SEQ ID NO: 1 or the nucleotide sequence of µ-conotoxin TIIIAlaMut SEQ ID NO: 4, both of which at the 5' end contain a sequence encoding six histidines (6His) linked by a serine-glycine-serine linker (SGS) to a construct encoding a TRX::TIIIA fusion protein containing the µ-conotoxin *TIIIA* gene and the gene encoding a leader protein, or a TRX::TIIIAlaMut fusion protein containing the µ-conotoxin *TIIIAlaMut* gene and a leader protein, where the said leader protein is thioredoxin (TRX) modified by point mutagenesis in which the amino acid methionine at position 37 has been replaced by lysine. Another subject matter of the invention is an expression vector containing the construct according to the invention under the control of a constitutive promoter. Another subject matter of the invention is an isolated *E. coli* cell containing the expression vector according to the invention. Another subject matter of the invention is a method for producing µ-conotoxin TIIIA or TIIIAlaMut in *E. coli* with the expression vector containing the construct according to the invention, characterized in that it includes the following steps: a) Transformation of an *E. coli* cell with the expression vector containing the construct according to the invention, under the control of a constitutive promoter, encoding either a TRX::TIIIA fusion protein with amino acid sequence SEQ ID NO: 2 or a TRX::TIIIAlaMut fusion protein with SEQ ID NO: 5; b) Expression of the TRX::TIIIA or TRX::TIIIAlaMut fusion protein; c) Isolation and purification of the TRX::TIIIA or TRX::TIIIAlaMut fusion protein; d) Disulfide bridges forming by subjecting the purified TRX::TIIIA or TRX:: TIIIAlaMut fusion protein to glutathione treatment in GSH/GSSG and dialysis in buffer; e) Cyanogen bromide cleavage of the TRX::TIIIA or TRX::TIIIAlaMut fusion protein with disulfide bridges formed; f) Purification of the cleaved TIIIA or TIIIAlaMut peptide.

## Description

Subject matter of the invention is a construct for an expression vector for recombinant µ-conotoxin TIIIA or TIIIAlaMut expression, expression vector containing the same, an isolated *E. coli* cell containing said expression vector, and a method for producing µ-conotoxin TIIIA or TIIIAlaMut in *E. coli* with expression vector containing said construct.

Conotoxins are substances with rapid paralyzing effects that, due to their properties, enable modulation of specific heterologous membrane receptors, ion channels and transporters. Conotoxins are found in nature in venom of sea snails of the *Conidae* family and are used to immobilize and paralyze prey. These snails inhabit coral reefs, and most are found in tropical and subtropical waters such as the South China Sea, the coast of Australia and the Pacific Ocean. There are about 700 species of cone snails and all of them are venomous. They are usually classified into three groups according to their feeding habits: worm hunters, mollusk hunters, and fish hunters. Conotoxins are small peptides derived from the cone snail venom that have evolved with the purpose to capture prey and defend against predators. They are structurally and functionally highly diverse class of bioactive molecules that are highly selective for various ligand- and voltage-gated ion channel subtypes. Conotoxins have proven to be a useful tool for studying the ligand-binding domain of ion channels. Moreover, they can be used as therapeutic agents to target specific ion channels. Ion channels are specialized cell membrane proteins that transport ions across the membrane. They are widely expressed in both excitable cells (neurons, muscles) and other cells (renal tubules, epithelial cells) (Gao B., Peng C., et al. (2017). Cone snail: A Big Store of Conotoxins for Novel Drug Discovery. Toxins 2017, 9,397; 1-19. doi:10.3390/toxins9120397 *Sillar* K.T., Picton L.D., Heilter W.J., (2016). The Neuroethology of Predation and Escape, First Edition, chapter 13. Neurotoxins for Attack and Defence. John Wiley & Sons, Ltd. Published*.* Oliviera B.M., Cruz L.J. (2001). Conotoxins, in retrospect. Toxicon 39; 7-14. doi:10.1016/50041-0101(00)00157-4)*.*

The mechanism of action of these peptides is blocking of specific neurotransmitters at nerve cell synapses. Conotoxins themselves, on the other hand, are microproteins usually less than 40 amino acid residues in length, which enables their heterologous, recombinant expression. The formation of (often multiple) disulfide bonds in a highly precise arrangement is particularly common among conotoxins and generally serves to stabilize the main bioactive conformation, critical for potency and selectivity, but also to enhance proteolytic resistance (Pennington M.W., Czerwinski A., Norton R.S. (2017). Peptide therapeutics from venom: Current status and potential. Bioorganic & Medical Chemistry 2017; doi:org/10.1016/j.bmc.2017.09.029)*.*

Venom of each individual cone snail contains up to 100 different peptides, each of which has a distinctive role when injected into the target. It is the cumulative effect of the individual peptides that makes the venom lethal to the prey. Based on their specificity, conotoxins have been grouped into several classes. Omega-conotoxins target and block voltage-sensitive Ca²⁺ channels, thereby inhibiting the neurotransmitter release. Alpha- and psi-conotoxins target and block nicotinic acetylcholine (ACh) receptors, causing ganglion and neuromuscular blockade. Mu (µ)- and delta-conotoxins block voltage-sensitive Na⁺ channels in muscle. Kappa-conotoxin blocks voltage-sensitive K⁺ channels, and these can also cause increased neuronal excitability. Gamma-conotoxin targets voltage-sensitive, non-specific cation channel, and sigma-conotoxin antagonizes the serotonin 5HT3 receptor (Becker S., Terlau H. (2008). Toxins from cone snails: properties, applications and biotechnological production. Microbiol. Biotechnology 79; 1-9. doi:10.1007/s00253-008-1385-6*;* Oliviera B.M. (1997). Conus venom peptides, receptor and ion channel targets and drug design: 50 million years of neuropharmacology. Mol Biol Cell 8; 2101-2109. doi:10.1091/mbc.8.11.2101*).*

Conotoxins are extremely specific biological probes that offer scientists a tool to understand and distinguish between closely related receptors. The simplicity of conotoxins has made them valuable in advancing neuroscience research, and consequently in development of drugs and cosmetic applications. Many diseases, such as epilepsy, schizophrenia, Tourette syndrome, Parkinson's disease and sclerosis, are associated with malfunctioning of ligand- and voltage-gated channels. Conotoxins have proven to be very promising because they are relatively small, potent, selective antagonists and agonists of specific cell membrane channel proteins, which can be used as great cosmetic and pharmaceutical tools (Mir R., Karim S., Mirza Z., et al. (2016). Conotoxins: Structure, Therapeutic Potential and pharmacological Applications. Current Pharmaceutical Design, 22; 582-589*;* Norton R.S., Oliviera B.M. (2006). Conotoxins down under. Toxicon 48; 780-798. doi:10.1016/j.toxicon.2006.07.022*).*

For example, the µ-CnIIIC conopeptide from the cone snail *Conus consors* exhibits myorelaxant activity through specific blockade of skeletal muscle Nav (Nav1.4) channels. This property can be used in cosmetics for daily anti-wrinkle therapy (Green B.R., Bulaj G., Norton R.S. (2014). Structure and function of µ-conotoxins, peptide-based sodium channel blockers with anglesis activity. Future Medicinal Chemistry 6(15); 1677-1698. doi:10.4155/FMC.14.107 Markgraf R., Leipold E., et al. (2012). Mechanism and molecular basis the sodium channel subtype specificity of µ-conopeptide CnIIIC. British Journal of Pharmacology 167; 576-586. doi:10.1111/j.1476-5381.2012.02004.x)*.*

Although conotoxins can be produced by various methods and techniques known in the art, currently the most common method for obtaining µ-conotoxins (mu-conotoxins) is their chemical synthesis (e.g., as indicated in patent EP1948685 B1). The second most common method is the conotoxin extraction from the venom of sea snails of the genus *Conus.* However, both methods involve a high cost of chemicals and protection of the necessary amino acids for synthesis. An economically viable alternative approach to producing such peptides in larger quantities appears to be the use of a bacterial expression system. In this context, *Escherichia coli* is the best characterized and most widely used bacterial host for the recombinant protein production.

The use of microbiological expression systems to produce conotoxins is in turn problematic due to the abundance of post-translational modifications in these peptides and the abnormal folding of the peptide form or accumulation of the protein in inclusion bodies in the cytoplasm of bacterial cells (Green BR, Bulaj G, Norton RS. Structure and function of µ-conotoxins, peptide-based sodium channel blockers with analgesic activity. Future Med. Chem. (2014) 6(15), 1677-1698*).*

Various research teams have tried to overcome the difficulties related with the use of microbiological expression systems. For example, patent EP2634252 B 1 relates to a method of expressing in *Escherichia coli* (*E. coli*) cells a eukaryotic protein that requires the formation of multiple interchain or intrachain disulfide bridges due to its biological activity. The said method includes: a) transformation of an *E. coli* cells with a first expression vector encoding the fusion protein, which contains an N-terminal segment encoding thioredoxin and a C-terminal segment encoding the eukaryotic protein, and a second expression vector encoding disulfide isomerase (DsbC) without its signal sequence (ΔssDsbC) and an alpha domain fragment of the thiol:disulfide interchange protein (DsbD); b) in which the host is deficient in one or more of the thioredoxin reductase (trxB), glutathione reductase (gor), ompT or Ion gene products; and c) expression of the said fusion protein. Moreover, the document in question reveals *E*. *coli* that is deficient in one or more of the thioredoxin reductase (*trxB*)*,* glutathione reductase (*gor*)*, ompT* or Ion gene products and is transformed with the first expression vector encoding a fusion protein that contains an N-terminal segment encoding thioredoxin, and a C-terminal segment encoding a eukaryotic protein, which requires the formation of multiple interchain or intrachain disulfide bridges, and a second expression vector encoding a disulfide isomerase (DsbC) that lacks a signal sequence (ΔssDsbC) and an alpha domain fragment of the thiol:disulfide interchange protein (DsbD).

In contrast, the 2013 publication by Klint *et al.* presents an optimized protocol for the expression of disulfide-rich venom peptides of predatory arthropods in the periplasm of *E. coli,* where the endogenous machinery for disulfide bond formation is located. Parameters studied include medium selection, induction conditions, lysis methods, purification methods for fusion proteins and peptides, and sample preparation for NMR studies (Klint JK, Senff S, Saez NJ, Seshadri R, Lau HY, Bende NS, Undheim EAB, Rash LD, Mobli M, King GF. Production of Recombinant Disulfide-Rich Venom Peptides for Structural and Functional Analysis via Expression in the Periplasm of E. coli. PLoS ONE 2013; 8(5): e63865. doi:10.1371/journal.pone.0063865)*.* Unfortunately, the method according to Klint *et al.* 2013, is based on expressing the protein into the periplasm to correctly assemble the disulfide bridges, a process which is difficult to control and involves the risk of disulfide bond destruction during the step of peptide isolation from the bacteria.

A publication by Nguyen *et al.* 2011 indicates in turn that it is possible to produce eukaryotic proteins with multiple disulfide bonds in the cytoplasm of *E. coli.* In addition, the required exogenous components can be introduced into a single plasmid vector, enabling easy transfer between different prokaryotic strains. Whereas the insertion of Ervlp, sulfhydryl oxidase and disulfide isomerase enables the efficient production of natively folded eukaryotic proteins with multiple disulfide bonds in the *E*. *coli* cytoplasm. The production of proteins with disulfide bonds was also supported by the use of a suitable fusion protein to maintain the solubility of the folding intermediates and by the selection of media. By combining the pre-expression of sulfhydryl oxidase and disulfide isomerase with these other factors, it is possible to obtain a high level expression of even complex disulfide bonded eukaryotic proteins (Nguyen VD, Hatahet F, Salo KEH, Enlund E, Zhang C, Ruddock LW. Pre-expression of a sulfhydryl oxidase significantly increases the yields of eukaryotic disulfide bond containing proteins expressed in the cytoplasm of E. coli. Microbial Cell Factories 2011, 10:1). In addition, this expression system requires gene expression induction with IPTG additive, which significantly increases the cost of the method. The method of Nguyen *et al.* 2013 also requires co-expression of enzymes that enable correct forming of disulfide bridges in the bacterial cytoplasm. Moreover, disulfide bridge formation in the cytoplasm involves the risk of disulfide bond destruction during the step of peptide isolation from the bacteria.

The objective of the invention is to develop a new efficient method for producing µ-conotoxin.

Subject matter of the invention is a construct for an expression vector for recombinant µ-conotoxin TIIIA or TIIIAlaMut expression, characterized in that it contains either the nucleotide sequence of µ-conotoxin TIIIA SEQ ID NO: 1 or the nucleotide sequence of µ-conotoxin TIIIAlaMut SEQ ID NO: 4, both of which at the 5' end contain a sequence encoding six histidines (6His) linked by a serine-glycine-serine linker (SGS) to a construct encoding either a TRX::TIIIA fusion protein containing the µ-conotoxin *TIIIA* gene and the gene encoding a leader protein, or a TRX::TIIIAlaMut fusion protein containing the µ-conotoxin *TIIIAlaMut* gene and a leader protein, where the said leader protein is thioredoxin (TRX) modified by point mutagenesis in which the amino acid methionine at position 37 has been replaced by lysine.

Another subject matter of the invention is an expression vector containing the construct according to the invention under the control of a constitutive promoter.

Preferably, the constitutive promoter is *deoP1P2.*

Preferably, the expression vector is pDM, preferably pDM with a nucleotide sequence SEQ ID NO: 7, or pDMR, preferably pDMR with a nucleotide sequence SEQ ID NO: 6.

Another subject matter of the invention is an isolated *E. coli* cell containing the expression vector according to the invention.

Preferably, the said cell is an *E. coli* S4B cell.

Another subject matter of the invention is a method for producing µ-conotoxin TIIIA or TIIIAlaMut in *E. coli* with the expression vector containing the construct according to the invention, characterized in that it includes the following steps:
a) Transformation of an *E. coli* cell with the expression vector containing the construct according to the invention, under the control of a constitutive promoter, encoding either a TRX::TIIIA fusion protein with amino acid sequence SEQ ID NO: 2 or a TRX::TIIIAlaMut fusion protein with SEQ ID NO: 5;
b) Expression of the TRX::TIIIA or TRX::TIIIAlaMut fusion protein;
c) Isolation and purification of the TRX::TIIIA or TRX::TIIIAlaMut fusion protein;
d) Disulfide bridges forming by subjecting the purified TRX::TIIIA or TRX::TIIIAlaMut fusion protein to glutathione treatment in GSH/GSSG and dialysis in buffer;
e) Cyanogen bromide cleavage of the TRX::TIIIA or TRX::TIIIAlaMut fusion protein with disulfide bridges formed;
f) Purification of the cleaved TIIIA or TIIIAlaMut peptide.

The constitutive promoter preferably used in step a) is *deoP1P2.*

The expression vector preferably used in step a) is pDM, preferably pDM with the nucleotide sequence SEQ ID NO: 7, or pDMR, preferably pDMR with the nucleotide sequence SEQ ID NO: 6.

Preferably, the transformed cell is the *E. coli* S4B cell.

Preferably, in step a) the transformation is carried out by electroporation.

Preferably, in step b) the culture of the transformed expressing *E. coli* strain is conducted at 30 °C.

Preferably, in step e), the cleavage is carried out using cyanogen bromide given in a molar excess of 100:1 relative to the methionine residues.

Preferably, in step e), the weight ratio of cyanogen bromide to the cleaved fusion protein is 1.375:1.

Preferably, in step e), the cleavage is carried out in 0.1M HCl for 3 hours at room temperature in the dark with stirring.

The invention provides the following benefits:
- In the method according to the invention, the recombinant conotoxin protein in soluble form is accumulated in the cytoplasm of the bacteria, while the disulfide bridges are formed *ex vivo* using glutathione (GSH/GSSG ratio). This enables to increase the control over the process and to avoid the risk of disulfide bond destruction during isolation of the peptide from the bacteria;
- The method according to the invention does not require induction with IPTG additive;
- The method uses a constitutive promoter that enables continuous synthesis of recombinant protein in bacterial cells and does not require induction for gene expression;
- The method according to the invention is characterized by high yield and generally lower cost compared to methods requiring disulfide bridge formation in the bacterial periplasm;
- The method according to the invention is characterized by a smaller number of steps than the methods known from the state of the art, which has a beneficial effect on the control of conditions in its individual steps;
- Conotoxins obtained by the method according to the invention will find wide application in the cosmetic industry.

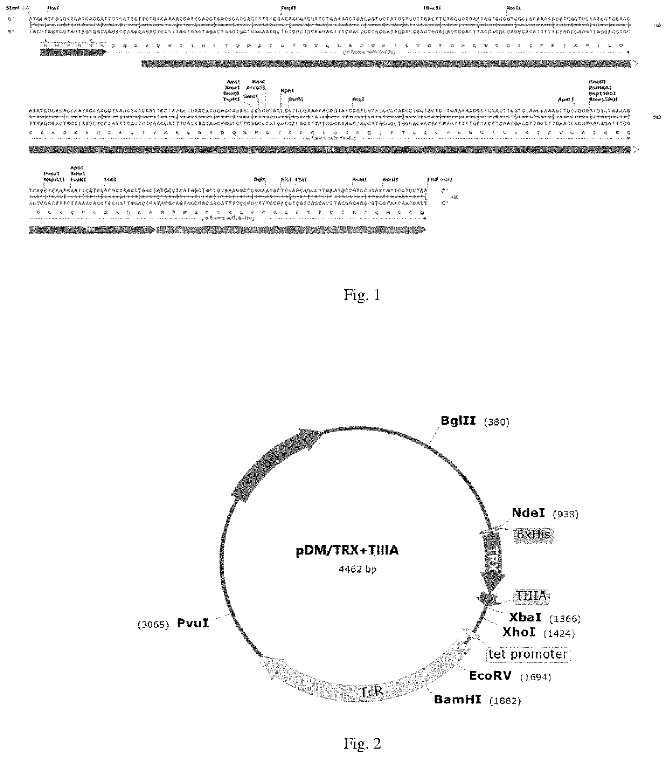

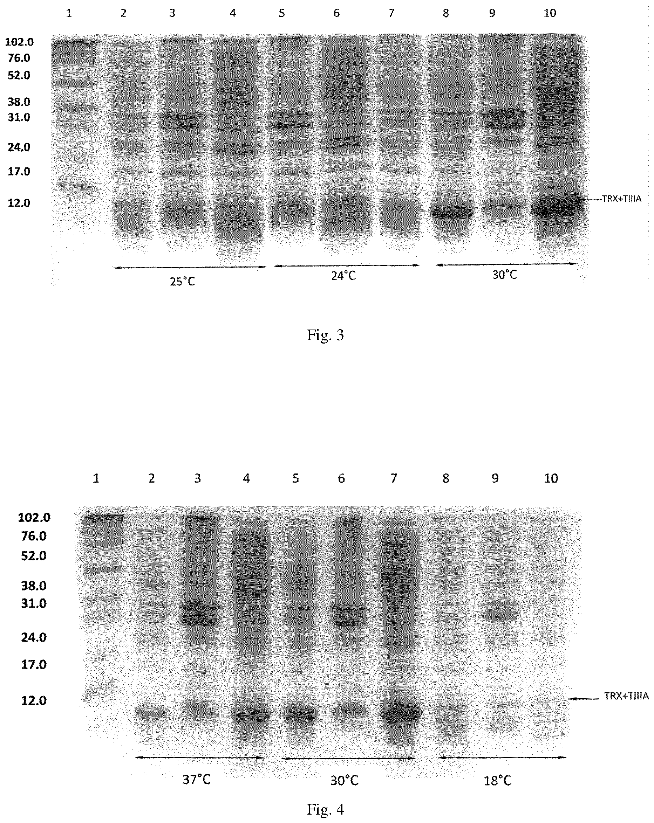

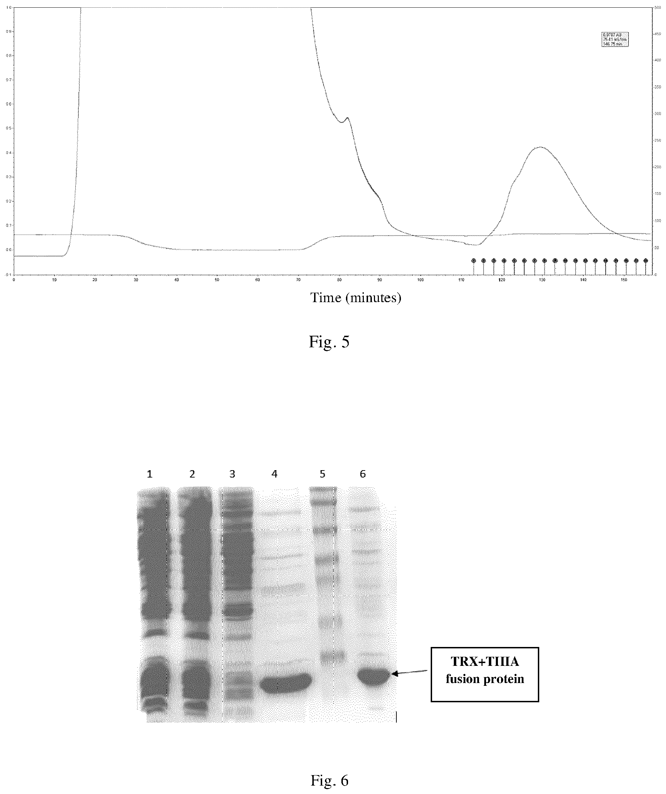

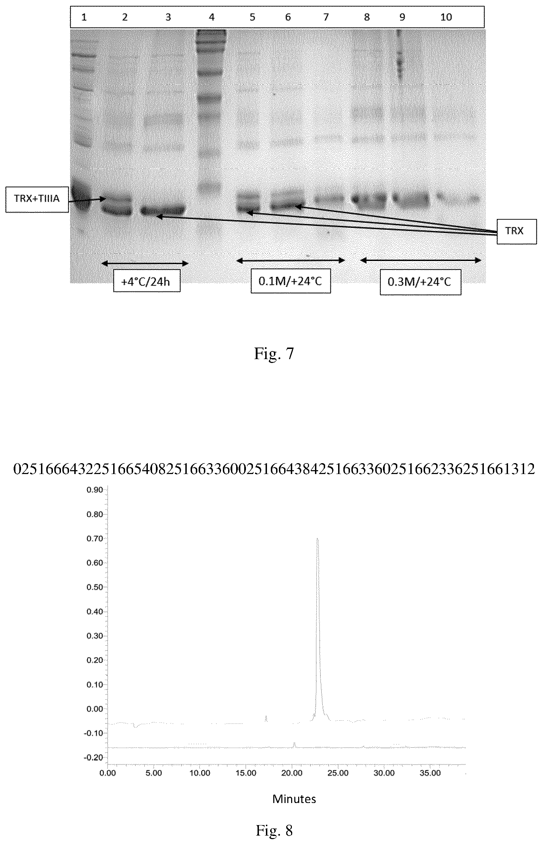

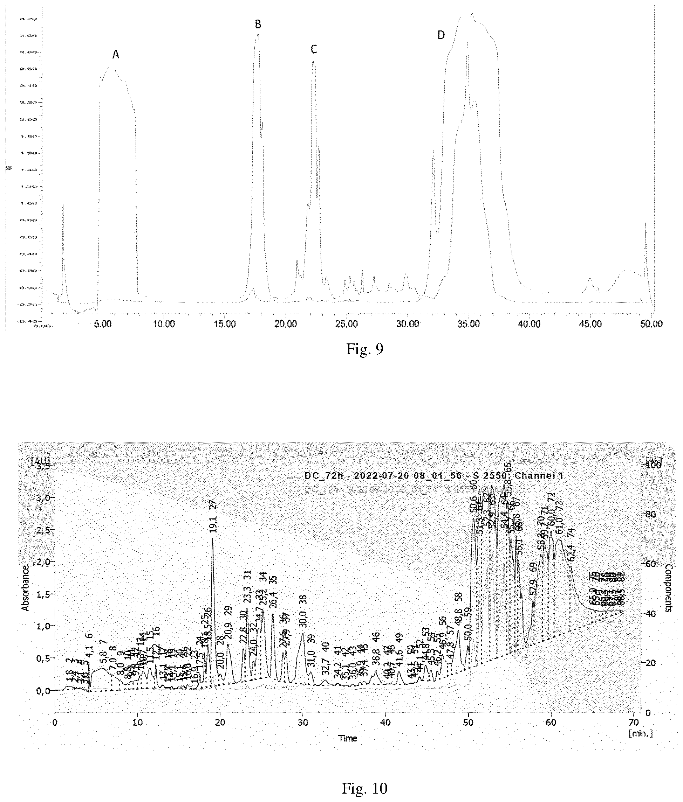

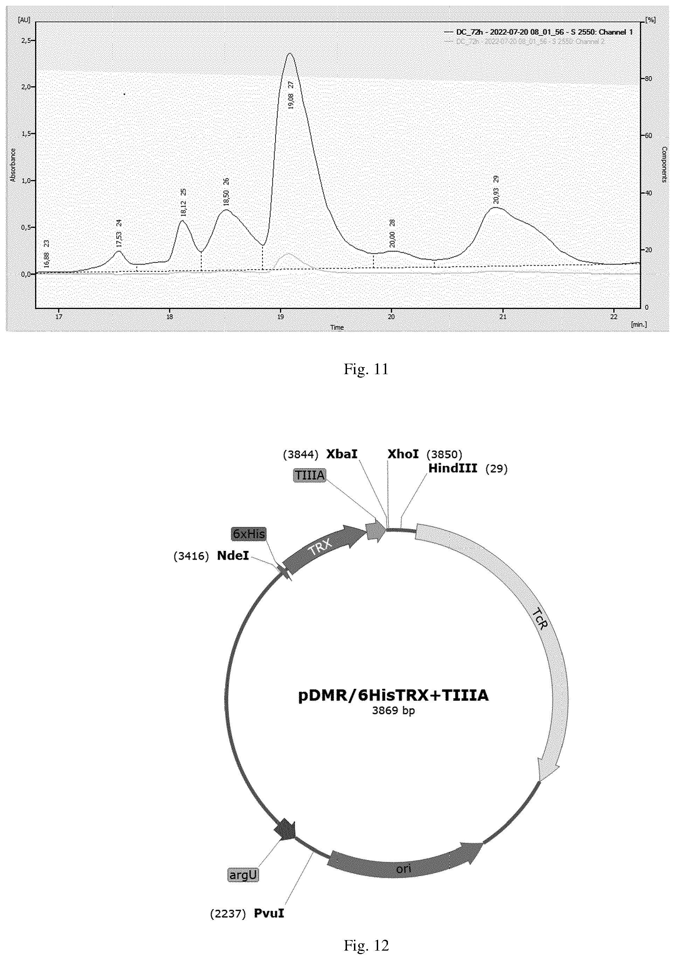

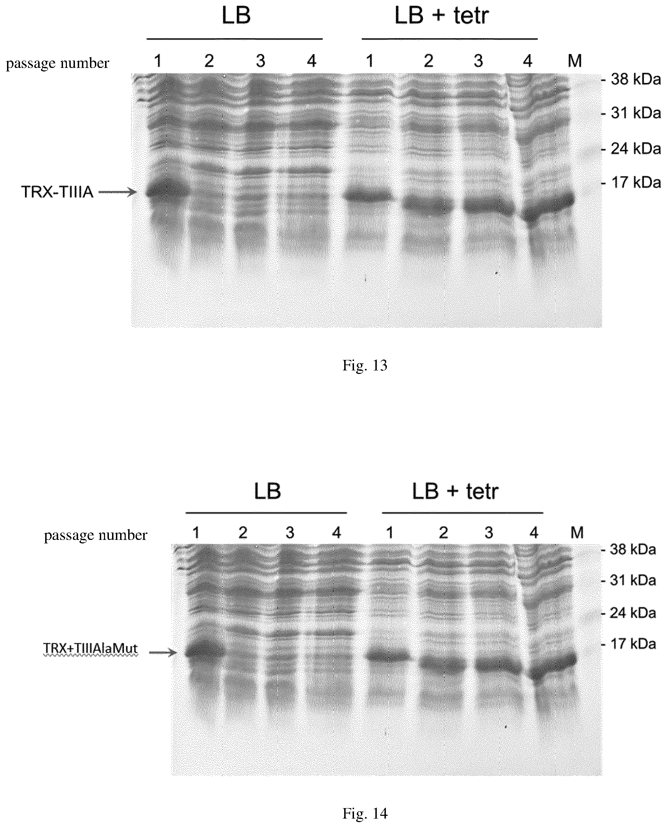

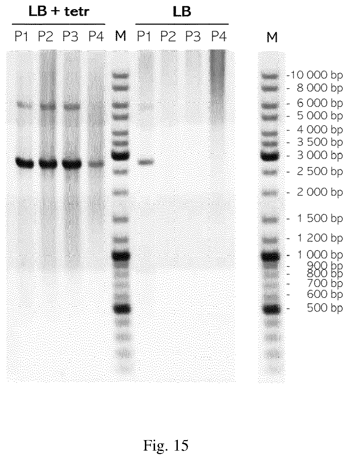

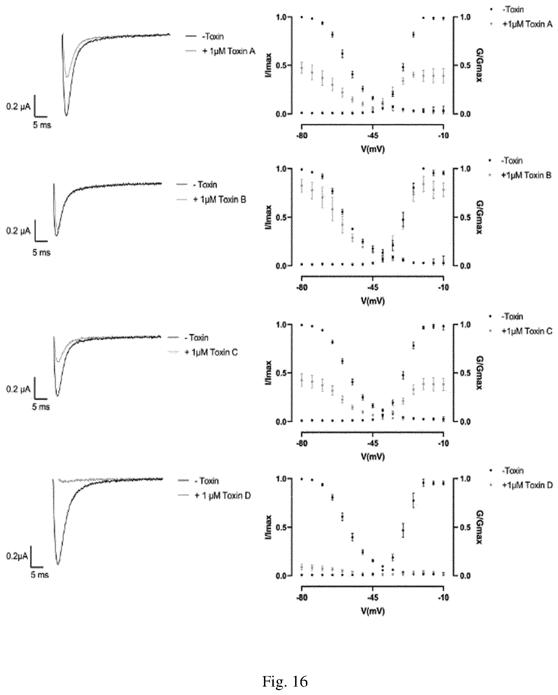

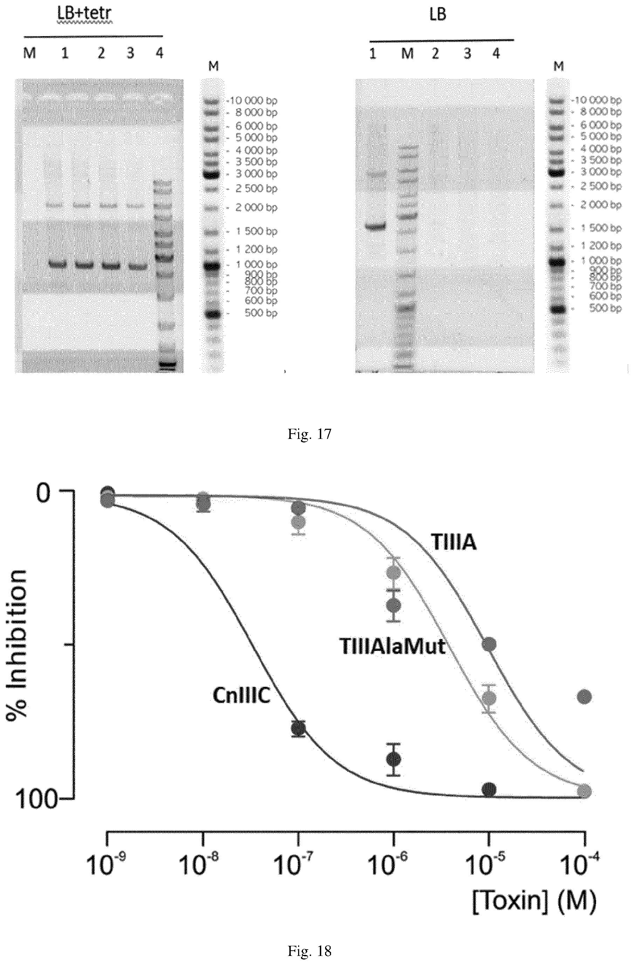

The invention in question is presented in examples of embodiment and in the drawing, in which: Fig. 1 schematically shows the nucleotide and amino acid sequences of the genetic construct according to the invention; Fig. 2 shows a schematic of the pDM/TRX+TIIIA construct used to produce recombinant conotoxin by the method according to the invention; Fig. 3 shows the electrophoretic separation of proteins of *E*. *coli* S4B transformed with a plasmid containing the *TRX*+*TIIIA* gene in SDS-PAGE (15% polyacrylamide gel), where 1 is the Full-Range Rainbow Amersham standard: 225; 150; 102; 76; 52; 38; 31; 24; 17; 12 kDa; 2 is a lysate of *E. coli* S4B/pDM/TRX+TIIIA, mass approximately 15 kDa from culture at 25 °C; 3 is a bacterial precipitate from culture at 25 °C after sonication; 4 is a supernatant from culture at 25 °C after sonication; 5 is a bacterial lysate of *E. coli* S4B/pDM/TRX+TIIIA from culture at 24 °C, 6 is a bacterial precipitate from culture at 24 °C after sonication, 7 is a supernatant from culture at 24 °C after sonication, 8 is a bacterial lysate of *E. coli* S4B/pDM/TRX+TIIIA from culture at 30 °C, 9 is a bacterial precipitate from culture at 30 °C after sonication, 10 is a supernatant from culture at 30 °C after sonication; Fig. 4 shows the electrophoretic separation of proteins of *E. coli* S4B transformed with a plasmid containing the *TRX*+*TIIIA* gene in SDS-PAGE (15% polyacrylamide gel), where 1 is the Full-Range Rainbow Amersham standard: 225; 150; 102; 76; 52; 38; 31; 24; 17; 12 kDa, 2 is a lysate of *E. coli* S4B/pDM/TRX+TIIIA; mass approximately 15 kDa from culture at 37 °C, 3 is a bacterial precipitate from culture at 37 °C after sonication, 4 is a supernatant from culture at 37 °C after sonication, 5 is a lysate of *E. coli* S4B/pDM/TRX+TIIIA from culture at 30 °C, 6 is a bacterial precipitate from culture at 30 °C after sonication, 7 is a supernatant from culture at 30 °C after sonication, 8 is a bacterial lysate of *E. coli* S4B/pDM/TRX+TIIIA from culture at 18 °C, 9 is a bacterial precipitate from culture at 18 °C after sonication, 10 is a supernatant from culture at 18 °C after sonication; Fig. 5 shows a chromatogram of *E. coli* transformed with the vector according to the invention; Fig. 6 shows electrophoretic separation of proteins of *E. coli* cultured at 30 °C transformed with a plasmid containing the *TRX*+*TIIIA* gene in SDS-PAGE (15%), where 1 is a bacterial lysate of *E. coli* S4B/pDM/TRX+TIIIA, mass approximately 14.4 kDa, 2 is a supernatant after sonication, 3 are bacterial proteins not immobilized on Ni-NTA bed, 4 are proteins after elution with elution buffer, 5 is the Full-Range Rainbow Amersham standard: 225; 150; 102; 76; 52; 38; 31; 24; 17; 12 kDa, 6 is a sample after dialysis after 48 hours (TRX::TIIIA fusion protein); Fig. 7 shows the electrophoretic separation of proteins after BrCN cleavage of TRX::TIIIA fusion protein in SDS-PAGE (15% polyacrylamide gel), where 1 is uncleaved TRX+TIIIA, 2 is 0.1M HCl cleavage after 24 hours at +4 °C BrCN 100:1, 3 is 0.3M HCl cleavage after 24 hours at +4 °C BrCN 100:1, 4 is the Full-Range Rainbow Amersham standard: 225; 150; 102; 76; 52; 38; 31; 24; 17; 12 kDa, 5 is 0.1M HCl cleavage after 3 hours at +24 °C BrCN 100:1, 6 is 0.1M HCl cleavage after 6 hours at +24 °C BrCN 100:1, 7 is 0.1M HCl cleavage after 24 hours at +24 °C BrCN 100:1, 8 is 0.3M HCl cleavage after 3 hours at +24 °C BrCN 100:1, 9 is 0.3M HCl cleavage after 6 hours at +24 °C BrCN 100:1, 10 is 0.3M HCl cleavage after 24 hours at +24 °C BrCN 100:1; Fig. 8 shows the spectrum of synthetic conotoxin TIIIA without disulfide bridges; Fig. 9 shows the spectra of the reaction mixture enriched with a synthetic fully reduced peptide (C), where peak A is the signal of low-mass organic compounds, peak B is the TIIIA with disulfide bridges, peak C is a synthetic admixed TIIIA without disulfide bridges, peak D is a carrier protein of 12.85 kDa and an uncleaved construct of 15.395 kDa; Fig. 10 shows an RP-HPLC chromatogram in the preparative phase, where the signal at 19.1 min corresponds to TIIIA, the signals from 21 to 50 min correspond to some cleavage of the targets and/or purification of the His tag, the peaks in the range of 50 to 56 min are the 12.8 kDa carrier protein, and 57-63 min is the uncleaved construct; Fig. 11 shows a section of the chromatogram from Fig. 10, with particular emphasis on the enlargement related to 17-22 min retention time, where the signal from 18.12 to 20.00 min corresponds to 3 mg of TIIIA; Fig. 12 shows a schematic of the pDMR/6HisTRX+TIIIA construct used to produce recombinant conotoxin by the method according to the invention; Fig. 13 shows the expression level of the TRX::TIIIA fusion protein in the total bacterial lysate after each passage, SDS-PAGE, 15% acrylamide, where the arrow points to a band indicating the expression (or lack thereof) of the desired fusion protein; Fig. 14 shows the expression level of TRX::TIIIAlaMut fusion protein in the total bacterial lysate after each passage, SDS-PAGE, 15% acrylamide, where the arrow points to a band indicating the expression (or lack thereof) of the desired fusion protein; Fig. 15 shows the material after pDM/TRX+TIIIA plasmid isolation after each passage, where Tetr: tetracycline, P1-P4: passage number, M: molecular marker; Fig. 16 shows the material after pDM/TRX+TIIIAlaMut plasmid isolation after each passage, where Tetr: tetracycline, 1-4: passage number, M: molecular marker; Fig. 17 shows the results of conotoxin activity measurements by patch-clamp on an oocyte model expressing human Nav 1.4 sodium channels for conotoxin TIIIA (Toxin A), conotoxin SIIIA (Toxin B), conotoxin TIIIAlaMut (Toxin C), CnIIIC standard (Toxin D), where the left column shows individual Nav 1.4 ion channel responses as an example of the observed inhibitory effect, while the right column shows the effect of the test substance on the conductance curve in function of the voltage and channel accessibility-two important parameters of Nav channel function that are usually evaluated; Fig. 18 shows the dose-response curve for TIIIA toxin, TIIIAlaMut toxin, and the standard-a commercially available CnIIIC toxin; where the effects were measured on the human NaV1.4 ion channel, and the data were fitted to a dose-response curve (Hill equation assuming a single binding site), with fitting performed using Graphpad Prism 8, with fixed parameters: Top = 0, Bottom = 99, Hill N = -1. Error bars represent S.E.M.

### Detailed description of the invention

Within the meaning of the invention, the phrase "in embodiment" is to be understood as in one or more embodiments. In addition, the features present in individual embodiments, including "some embodiments," may be combined with each other. The descriptions of embodiment of the invention in this application are given by way of example and are not intended to limit the scope of the invention. The described embodiments include various features, not all of which are required in all embodiments of the invention. Some embodiments use only some of the features or possible combinations of features. The described variants of the embodiments of the invention, as well as embodiments of the invention including various combinations of the features mentioned in the described embodiments will be appreciated by those skilled in the art. The scope of the invention is limited only by the claims.

In a first aspect, the invention relates to a construct for an expression vector for recombinant µ-conotoxin TIIIA or TIIIAlaMut expression that contains either the nucleotide sequence of µ-conotoxin TIIIA SEQ ID NO: 1 or the nucleotide sequence of µ-conotoxin TIIIAlaMut SEQ ID NO: 4, both of which at the 5' end contain a sequence encoding six histidines (6His) linked by a serine-glycine-serine linker (SGS) to a construct encoding either a TRX::TIIIA fusion protein containing the µ-conotoxin *TIIIA* gene and the gene encoding a leader protein, or a TRX::TIIIAlaMut fusion protein containing the µ-conotoxin *TIIIAlaMut* gene and a leader protein, where the said leader protein is thioredoxin (TRX) modified by point mutagenesis, in which the amino acid methionine at position 37 has been replaced by lysine.

The nucleotide sequence of either SEQ ID NO: 1 or SEQ ID NO: 4, both of which at the 5' end contain a sequence encoding six histidines (6His) linked by a serine-glycine-serine linker (SGS) to a construct encoding a TRX::TIIIA or TRX::TIIIAlaMut fusion proteins containing the conotoxin *TIIIA* gene or TIIIAlaMut gene and the gene encoding the leader protein, which is thioredoxin (TRX) modified by point mutagenesis, in which the amino acid methionine at position 37 has been replaced by lysine.

In one embodiment of the construct according to the invention, it contains the nucleotide sequence of µ-conotoxin TIIIA SEQ ID NO: 1. In another embodiment, the construct contains the nucleotide sequence of µ-conotoxin TIIIAlaMut SEQ ID NO: 4.

The construct according to the invention is under the control of a constitutive promoter, which enables continuous synthesis of the recombinant protein in bacterial cells and does not require induction for gene expression.

In some embodiments, the constitutive promoter is the *deoP1P2* constitutive promoter.

The *deoP1P2* promoter (NCBI GenBank accession number: AP009048) is derived from a bacterial deo operon composed of four closely related genes that regulate nucleotide and deoxynucleotide catabolism in *Escherichia coli.* (Valentin-Hansen, P., Hammer, K., Larsen, J.E., Svendsen, I. (1984) The internal regulated promoter of the deo operon of Escherichia coli K-12. Nucleic Acids Research 12(13), 5211-5224. Doi: 10.1093/nar/12.13.5211; P. Valentin-Hansen, H. Aiba, D. Schümperli. The structure of tandem regulatory regions in the deo operon of Escherichia coli K12, The EMBO Journal (1982)1:317-322 https://doi.org/10.1002/j.1460-2075.1982.tb01167.x; G. Dandanell, K. Hammer. Two operator sites separated by 599 base pairs are required for deoR repression of the deo operon of Escherichia coli. The EMBO Journal (1985)4:3333-3338https://doi.org/10.1002/j.1460-2075.1985.tb04085.x).

In a further aspect, the invention relates to an expression vector containing a construct according to the invention under the control of a constitutive promoter.

In one embodiment, the construct according to the invention contained in the expression vector contains the nucleotide sequence SEQ ID NO: 1.

In another embodiment, the construct according to the invention contained in the expression vector is the nucleotide sequence SEQ ID NO: 4.

In some embodiments, the constitutive promoter is d*eoP1P2.*

In some embodiments, the expression vector is pDM expression vector. In a preferred embodiment, the pDM expression vector has the nucleotide sequence SEQ ID NO: 7.

In some embodiments, the expression vector is pDMR.

In preferred embodiments, the expression vector is pDMR having the nucleotide sequence SEQ ID NO: 6.

In a further aspect, the invention relates to an isolated *E. coli* cell containing the expression vector according to the invention. In some embodiments, said isolated cell is an *E. coli* S4B cell, in particular *E. coli* S4B, which is a derivative of the reference *E. coli* strain. In one embodiment, the *E. coli* S4B strain is a derivative of the *E. coli* DH10B strain. In another embodiment, the *E. coli* S4B strain is a derivative of the *E. coli* JM109 strain. In another embodiment, the *E. coli* S4B strain is a derivative of the *E. coli* ATCC 39111 strain. In another embodiment, the *E. coli* S4B strain is a derivative of the *E. coli* HB101 strain. In another embodiment, the *E. coli* S4B strain is a derivative of the *E. coli* CSH50R strain. In another embodiment, the *E. coli* S4B strain is a derivative of the *E. coli* BL21(DE3) strain.

In a further aspect, the invention relates to a method for producing µ-conotoxin TIIIA in *E. coli* with the expression vector containing the construct according to the invention, which includes the following steps:
a) Transformation of an *E. coli* cell with the expression vector containing the construct according to the invention, under the control of a constitutive promoter and encoding TRX::TIIIA fusion protein with amino acid sequence SEQ ID NO: 2 or TRX::TIIIAlaMut acid sequence SEQ ID NO: 5;
b) Expression of the TRX::TIIIA or TRX::TIIIAlaMut fusion protein;
c) Isolation and purification of the TRX::TIIIA or TRX::TIIIAlaMut fusion protein;
d) Disulfide bridges forming by subjecting the purified TRX::TIIIA or TRX::TIIIAlaMut fusion protein to glutathione treatment in GSH/GSSG and dialysis in buffer;
e) Cyanogen bromide cleavage of the TRX::TIIIA or TRX::TIIIAlaMut fusion protein with disulfide bridges formed;
f) Purification of the cleaved TIIIAor TIIIAlaMut peptide.

In one embodiment of the construct according to the invention used in step a) of the method according to the invention, it contains the nucleotide sequence SEQ ID NO: 1 and encodes a fusion protein of the amino acid sequence SEQ ID NO: 2.

In one embodiment of the construct according to the invention used in step a) of the method according to the invention, it contains the nucleotide sequence SEQ ID NO: 4 and encodes a fusion protein of the amino acid sequence SEQ ID NO: 5.

In some embodiments, the constitutive promoter used in step a) is *deoP1P2.*

In some embodiments, the expression vector used in step a) is pDM. In a preferred embodiment, the pDM expression vector has the nucleotide sequence SEQ ID NO: 7.

In other embodiments, the expression vector is pDMR.

In some preferred embodiments, the expression vector is pDMR with the nucleotide sequence SEQ ID NO: 6.

In some embodiments, the cell transformed in step a) is an *E. coli* S4B cell, which is a derivative of the reference *E. coli* strain. In addition, in one embodiment, the *E. coli* S4B strain is a derivative of the *E. coli* DH10B strain. In another embodiment, the *E. coli* S4B strain is a derivative of the *E. coli* JM109 strain. In another embodiment, the *E. coli* S4B strain is a derivative of the *E. coli* ATCC 39111 strain. In another embodiment, the *E. coli* S4B strain is a derivative of the *E. coli* HB101 strain. In another embodiment, the *E*. *coli* S4B strain is a derivative of the *E. coli* CSH50R strain. In another embodiment, the *E. coli* S4B strain is a derivative of the *E. coli* BL21(DE3) strain.

In some embodiments, the transformation in step a) is carried out by electroporation.

In one embodiment, in step b) of the method according to the invention, the culture of the transformed expressing *E. coli* strain is conducted at 30 °C, in particular to obtain an optical density of OD₆₀₀≈1.0 In contrast, in other embodiments other temperatures were used, in particular 18 °C, 25 °C or 37 °C to obtain an optical density of OD₆₀₀≈1.0.

In some embodiments, in step e) of the method according to the invention, the cleavage is carried out using cyanogen bromide given in a molar excess of 100:1 relative to the methionine residues.

In one embodiment, in step e), the weight ratio of cyanogen bromide to the cleaved fusion protein is 1.375:1.

In some embodiments, the cleavage in step e) is carried out with the addition of HCl. In one embodiment, the cleavage in step e) is carried out with the addition of 0.1M HCl for 3 hours at room temperature in the dark with stirring. In one embodiment, the room temperature is defined as 20-24 °C.

In another embodiment, the cleavage is carried out with the addition of 0.1M HCl for 24 hours at +4 °C. In another embodiment, the cleavage is carried out with the addition of 0.3M HCl for 24 hours at +4 °C.

In another embodiment, the cleavage is carried out with the addition of 0.1M HCl for 24 hours at room temperature, in particular at +24 °C. In another embodiment, the cleavage is carried out with the addition of 0.1M HCl for 6 hours at +4 °C. In another embodiment, the cleavage is carried out with the addition of 0.3M HCl for 3 hours at room temperature, in particular at +24 °C. In another embodiment, the cleavage is carried out with the addition of 0.3M HCl for 24 hours at room temperature, in particular at +24 °C.

The invention is presented in examples of embodiment, and all tests and experimental procedures described below were carried out using commercially available test kits, reagents and equipment, following the manufacturer recommendations of the kits, reagents and equipment used, unless specifically indicated otherwise. All test parameters were measured using standard, well-known methods used in the art to which the present invention pertains.

At the same time, the following examples of embodiment serve to illustrate the solution in question and should not constitute a limitation of the scope of patent protection.

### Example 1

### Designing E. coli bacterial expression system with a pDM/TRX+TIIIA construct

In order to obtain a recombinant soluble conotoxin TIIIA protein, a genetic construct has been designed, which is a construct according to the invention encoding the TRX::TIIIA fusion protein, containing a conotoxin *TIIIA* gene and a gene encoding thioredoxin (*TRX*) which is the leader protein. The TRX protein provides reducing conditions to facilitate the correct folding of proteins having disulfide bridges, moreover, it also often leads to the expression of recombinant proteins in soluble form, which in this case significantly shortens the purification process of the recombinant protein. In addition, the nucleotide sequence of the *TRX* gene was modified using a point mutagenesis reaction, by which the amino acid methionine (M) at position 37 was removed and replaced with lysine (K). This modification was to obtain the correct protein fragments after cyanogen bromide (BrCN) cleavage, whose cleavage site is methionine. The nucleotide sequence of the *TRX::TIIIA* fusion gene was optimized for bacterial codon usage. In addition, the construct according to the invention is under the control of a constitutive promoter, preferably *deoP1P2*, which enables continuous synthesis of the recombinant protein in bacterial cells and does not require induction for gene expression.

To enable purification of the protein on a chromatographic column with a Ni-NTA bed, a sequence encoding 6 histidines (6His) and a short linker including the amino acids serine-glycine-serine (SGS) were added to the 5' end of the construct. The TRX::TIIIA fusion protein gene was inserted into the expression vector according to the invention, which in this example of embodiment is the pDM expression vector cleaved with NdeI/XbaI restriction enzymes.

The nucleotide sequence (SEQ ID NO: 1) and amino acid sequence (SEQ ID NO: 2) of the genetic construct according to the invention are shown in Fig. 1.

The nucleotide sequence of the cloned genes was confirmed, and a production strain according to the invention was constructed, which in this example of embodiment is an *E. coli* S4B production strain that is a derivative of *E. coli* DH10B strain containing the pDM/TRX+TIIIA construct (a single molecule of conotoxin TIIIA combined with thioredoxin) to produce a soluble peptide. In addition, the *E. coli* S4B production strain may be a derivative of other *E. coli* strains, particularly of JM109, HB101, CSH50R, and DH10B strains.

The procedure was carried out analogously for a construct according to the invention encoding the TRX::TIIIAlaMut fusion protein with nucleotide sequence SEQ ID NO: 4 and amino acid sequence SEQ ID NO: 5.

### Example 2

### Designing E. coli bacterial expression system with a pDMR/TRX+TIIIA construct

The design of the *E. coli* bacterial expression system was carried out as in Example 1, except that an expression vector referred to as pDMR was used, which is a pBR322 vector derivative and contains the constitutive promoter *deoP1P2*, the tetracycline resistance gene, the *tRNA-Arg* gene encoding the UCU/CCU anticodon, and the sequence of the TRX::TIIIA fusion protein (pDMR/6HisTRX+TIIIA SEQ ID NO: 6). The *tRNA-Arg* gene makes up for the deficiency of rare arginine codons in bacteria. The introduction of an additional pool of such tRNA molecules may increase the yield of heterologous protein synthesis in *E. coli.*

### Example 3

The method for producing µ-conotoxin TIIIA in *E. coli* with an expression vector containing the construct according to Example 1 includes the following steps:
**Step a)** *Transformation of an E. coli cell with the expression vector containing the construct according to the invention*

In this example of embodiment, a prokaryotic pDM/TRX+TIIIA expression vector containing a construct with the nucleotide sequence SEQ ID NO: 1 encoding the TRX::TIIIA fusion protein with the amino acid sequence SEQ ID NO: 2 was inserted by electroporation into an expressing *E. coli* S4B strain being a derivative of a reference *E. coli* strain (e.g., *E. coli* DH10B, but *E. coli* JM109, *E. coli* HB101, *E. coli* CSH50R, *E. coli* BL21(DE3) can also be used).

In addition, in this example of embodiment, the construct according to the invention is under the control of a constitutive promoter, preferably *deoP1P2*, which enables continuous synthesis of the recombinant protein in bacterial cells and does not require induction for gene expression.

A schematic of the pDM/TRX+TIIIA construct used to produce the recombinant conotoxin by the method according to the invention is shown in Fig. 2.

### Step b) Expression of the TRX: : TIIIA fusion protein in a transformed E. coli strain

In order to verify that the resulting protein is in soluble form and to select culture conditions leading to the highest possible expression of the gene encoding the recombinant TRX::TIIIA fusion protein, liquid LB medium supplemented with tetracycline (100 µg/mL) was inoculated using material from a single colony of a transformed *E. coli* strain, and cultured at various temperatures of 18 °C, 25 °C, 30 °C, 37 °C until an optical density of OD₆₀₀≈1.0 was achieved. The results are shown in Figs. 3-4.

The conducted analysis showed that the recombinant protein is obtained in soluble form, while the highest expression of the gene encoding the TRX::TIIIA fusion protein is obtained at 30 °C.

Then, in order to obtain the optimal expression of the conotoxin TIIIA on a laboratory scale, the transformed *E*. *coli* strains were cultured in LB medium (10 g/L Bacto Tryptone, 5 g/L yeast extract, 10 g/L NaCl) with the addition of the selection marker tetracycline (100 µg/mL), at 30 °C, at 150 rpm for 18 hours, achieving an OD of 3.2 to 3.5. Culture media were inoculated with a suitable volume of culture material (stock) stored at -70 °C (500 µL stock per 500 mL LB medium). Stocks were prepared with bacterial culture in a 1:1 ratio (OD₆₀₀≈0.6) and 20% glycerol. Bacterial material is deposited in the Applicant's strain bank.

### Step c) Isolation and purification of TRX::TIIIA fusion protein

After 18 hours, the culture prepared in step c) and conducted at 30 °C was centrifuged (8000 rpm), and the centrifuged biomass from 1 L of the culture was resuspended in 50 mL of prepared dissolving buffer (50 mM TRIS-HCl pH 7.8; 300 mM NaCl), sonicated for 1 hour 15 min, centrifuged twice for 15 min at 12,000 rpm. The supernatant was loaded onto a column previously equilibrated with calibration buffer. The bed was then washed with wash buffer and the recombinant TRX::TIIIA protein was eluted with elution buffer. The flow rate, when loading the sample, was 1.0 mL/min, the column was washed at a flow rate of 1.5 mL/min, and the elution was carried out at a flow rate of 2.0 mL/min.

The following buffers were used for protein purification:
- Calibration buffer
   ∘ 50 mM phosphate buffer, pH 7.8, 500 mM NaCl, 10 mM imidazole;
- Wash buffer
   ∘ 50 mM phosphate buffer, pH 7.8, 500 mM NaCl, 20 mM imidazole;
- Elution buffer
   ∘ 50 mM phosphate buffer, pH 7.8, 500 mM NaCl, 150 mM imidazole;
5 mL of each fraction was collected. Separation was carried out on a Bio-Rad Duo Flow System instrument using a column from the same company. The concentration of the recombinant TRX::TIIIA protein was determined using the Bradford method: 10 µL of sample, 990 µL of 20 mM Tris-HCl buffer pH 7.6, 1 mL of Bradford reagent. Absorbance was read on a spectrophotometer at 595 nm. The result of LPLC chromatographic separation from 1 L of bacterial culture expressing TRX::TIIIA protein, on SuperFlow Ni-NTA affinity bed, is shown in Fig. 5.

### Step d) Disulfide bridge forming by subjecting the purified TRX::TIIIA fusion protein to glutathione treatment in GSH/GSSG and dialysis in buffer

After chromatographic separation, about 30-35 mL of eluate with a protein concentration of about 1.0 mg/mL was obtained from 1 L of culture. After elution, up to 4 mM of reduced glutathione (GSH) and up to 1 mM of oxidized glutathione (GSSG) were added to the collected fractions, in order to obtain correct disulfide bridge formation. The sample thus prepared was dialyzed in buffer (50 mM TRIS-HCl buffer pH 7.8, 10% glycerol). Dialysis was carried out for 48 hours at 4 °C; after 24 hours, the buffer was changed.

The composition of the obtained fractions was evaluated by polyacrylamide gel electrophoresis (SDS-PAGE). Protein electrophoresis under denaturing conditions of SDS-PAGE was used to analyze the purity of the obtained recombinant TRX::TIIIA protein obtained in the prokaryotic system. The obtained results are shown in Fig. 6.

### Step e) Cyanogen bromide cleavage of the TRX: : TIIIA fusion protein with disulfide bridges formed

Cyanogen bromide (BrCN) cleavage of proteins occurs in an acidic environment. The cleavage reaction is highly specific, BrCN reacts with the sulfur of the side chain of the amino acid methionine (Met) residue causing the peptide bond to break after the carboxyl moiety of methionine, which is one of the rarest amino acids in proteins.

In order to cleavage the recombinant conotoxin off the leader protein, thioredoxin, it was decided to cleavage with BrCN given in a molar excess of 100:1 (100 mole of BrCN per 1 Met residue) in acidic medium. The nucleotide sequence of the TRX::TIIIA fusion protein contains two methionines followed by BrCN cleavage.

The amount of BrCN required for the reaction per methionine residue was calculated based on the molar mass of the fusion protein:

| | |
|---|---|
| Fusion protein: | TRX::TIIIA |
| molar mass: | 15,403.67 g/mol |
| excess of BrCN relative to methionine residues: | 100:1 |

For 1 mg of fusion protein, take 1.375 mg of BrCN. This amount is contained in 2.60 µL of a 5M solution of BrCN in CH₃CN (d=1.093).

TRX::TIIIA fusion protein at a concentration of 1.0 mg/mL, obtained after chromatographic separation and 48-hour dialysis, was cleaved with BrCN with the addition of various concentrations of HCl, at +4 °C, and at room temperature in the dark with stirring. Samples cleaved at room temperature were collected after 3, 6 and 24 hours to determine the most productive cleavage time.

Samples after cleavage were handed over for LC-MS (Liquid Chromatography-Mass Spectrometry) study.

Protein electrophoretic separation after BrCN cleavage of the TRX::TIIIA fusion protein in SDS-PAGE (15% polyacrylamide gel) (Fig. 7) showed that the most optimal cleavage conditions for the TRX::TIIIA fusion protein at a BrCN molar excess of 100:1 in acidic medium were 0.1 M HCl, three hours at room temperature, in the dark with stirring.

### Step f) Purification of the cleaved TIIIA peptide

In this example of embodiment, purification of the cleaved TIIIA peptide was carried out by RP-HPLC. After BrCN cleavage, mainly two molecules were obtained from the TRX::TIIIA fusion protein with a molecular weight (MW) of 15,395 Da:
- TIIIA peptide with a molecular weight of 2426.836 Da,
- TRX leader protein with a molecular weight of 12,855 Da.

Other nonspecific products are present in variable proportions.

To test the properties of a fully reduced peptide without disulfide bridges, a linear peptide identical to the sequence of the conotoxin TIIIA (SEQ ID NO: 3) was synthesized. The linear peptide is more hydrophobic than the peptide containing disulfide bridges, and has a longer retention time compared to the correctly folded peptide, as shown in Figs. 8-9.

For the quality control (QC) shown in Figs. 8-9 an analytical/semi-preparative HPLC kit (Waters) was used.

Separation parameters:
- C18 column 250x10mm, 100 Å, 5 µm
- buffer A: 0.1% TFA in water
- buffer B: 90% MeCN, 0.1% TFA, water
- MeCN gradient 5-100% buffer B,
- flow rate 2 mL/min, 5-100% buffer B in 55 min.

For the purification process of the TIIIA peptide, shown in Figs. 10-11, a preparative HPLC system was used (Knauer).

Separation parameters:
- C18 column 250x21.2 mm, 100 Å, 5 µm
- buffer A: 0.1% TFA in water
- buffer B: 90% MeCN, 0.1% TFA, water
- MeCN gradient 5-100% buffer B,
- flow rate 20 mL/min, 5-100% buffer B in 70 min.

One of the following two procedures can be used for purification:
Procedure I

The post-BrCN cleavage mixture was divided into portions of 25 mL each in 50 mL conical vials, frozen in liquid nitrogen and lyophilized at -80 °C, vacuum -3 mBar. The lyophilization time depends on the total volume of solvent to be removed and varies from 10 to 30 hours. The dry residues after evaporation of 25 mL of reaction mixtures were dissolved in 2 mL of 0.1% TFA, 5% MeCN, centrifuged at 15 kG for 20 minutes at room temperature. Samples were injected into the HPLC, using a 2-mL loop for semi-preparative separation and a 5-mL loop for preparative separation. Samples were separated in MeCN A-B gradient, 5-100% in the presence of 0.1% TFA. The fraction containing TIIIA was collected, frozen in liquid nitrogen and lyophilized. The dry sample was weighed.

Separation on a preparative system results in the detection of a greater number of different signals due to the significant amount of the loaded material. However, this does not affect the correct separation of conotoxin TIIIA.

Due to the different column size and gradient length, separation of the uncleaved construct and carrier proteins occurs. Proper scaling-up (>100 mg) of the method requires the introduction of additional preparatory steps.

### Procedure II

Due to the unfavorable ratio (1:6) of the TIIIA peptide (2.4 kDa) to the mass of the entire TRX::TIIIA construct (15.3 kDa) and the resulting second TRX product (12.8 kDa) after BrCN cleavage, and due to the limited volume of HPLC columns, it was better to remove the uncleaved protein and second product before the actual HPLC separation. A 10g cartridge with a C18 bed designed for FPLC chromatography was used to bind the peptides and proteins present in the mixture after cleavage. Salts and other small molecules were removed during the washing step with 2% MeCN, 0.1% TFA. The crude fraction containing TIIIA was eluted with 30% MeCN, 0.1% TFA, frozen in liquid nitrogen and lyophilized, and then subjected to an HPLC protocol for final purification. The C18 cartridge was washed with 90% MeCN 0.1% TFA, followed by 80% MeOH, 0.1%. The fractions were checked for the presence of the TIIIA peptide and again subjected to the purification procedure. The results are shown in Figs. 10-11.

### Example 4

The method is as in Example 3, except that in step a) the pDM/TRX+TIIIAlaMut expression vector containing a construct with nucleotide sequence SEQ ID NO: 4 encoding the TRX::TIIIAlaMut fusion protein with amino acid sequence SEQ ID NO: 5 was used.

### Example 5

The method is as in Example 3, except that in step a) the expression vector pDMR/6HisTRX+TIIIA with the sequence SEQ ID NO: 6 was used. In addition, the said expression vector referred to as pDMR is a derivative of the pBR322 vector and contains the constitutive promoter *deoP1P2*, the tetracycline resistance gene, the *tRNA-Arg* gene encoding the UCU/CCU anticodon and the sequence of the TRX::TIIIA fusion protein. The *tRNA-Arg* gene makes up for the deficiency of rare arginine codons in bacteria. The introduction of an additional pool of such tRNA molecules may increase the yield of heterologous protein synthesis in *E. coli.*

A schematic of the pDMR/6HisTRX+TIIIA construct used to produce the recombinant conotoxin by the method according to the invention is shown in Fig. 12.

In addition, the above procedure was performed analogously with respect to the pDMR/6HisTRX+TIIIAlaMut plasmid.

### Example 6

The method is as in Example 3, except that in step a) the pDM expression vector with the sequence of ID SEQ NO: 7 was used, wherein pDM is a derivative of the pBR322 vector, contains the constitutive promoter *deoP1P2*, the tetracycline resistance gene, and the sequence of the TRX::TIIIA fusion protein.

### Example 7

### Analysis of plasmid stability in E. coli cells according to the invention producing the TRX::TIIIA fusion protein and in E. coli cells according to the invention producing the TRX::TIIIAlaMut fusion protein

### Methodology

The stability of the pDM/TRX+TIIIA plasmid in *E. coli* cells according to the invention was verified during four 22-hour passages. Bacteria were cultured in LB liquid medium (Luria-Bertani, Roth) in the presence of an antibiotic (tetracycline) and without it.

Passaging was carried out at a dilution of 1:1000 (100 µL of bacterial culture was suspended in 100 mL of fresh medium), with and without the presence of the selection marker tetracycline. Bacteria were cultured in LB medium at 30 °C, 150 rpm. After each passage, the optical density at λ = 600 nm (OD₆₀₀) was monitored, from which the number of generations per day (gn) was calculated. On the other hand, the total number of generations was calculated as the sum of the daily values.

To calculate the bacterial titer after each passage, the bacterial sample was plated onto LB agar plates with/without antibiotic. After plating onto the agar plates, the ratio of antibiotic-resistant to antibiotic-susceptible bacterial colonies was calculated. The pDM/TRX+TIIIA plasmid has a tetracycline resistance gene and a self-inducing promoter. The expression level of the fusion protein after each passage was verified on a polyacrylamide gel. Moreover, the plasmid was isolated from the bacterial sample after each passage.

The procedure was performed analogously with respect to the pDM/TRX+TIIIAlaMut plasmid.

### Results

The results of the OD₆₀₀ optical density measurements and the number of generations after each passage of *E*. *coli* cells with the pDM/TRX+TIIIA plasmid and with the pDM/TRX+TIIIAlaMut plasmid are shown in Tables 1 and 2, respectively.

**Table 1. OD₆₀₀ values for each passage of E. coli cells with pDM/TRX+TIIIA plasmid after 22 hours of incubation and number of generations**

| **Passage number** | **OD₆₀₀** | | **Number of generations** | | | |
|---|---|---|---|---|---|---|
| | | | **per day** | | **total** | |
| | **LB** | **LB + tet** | **LB** | **LB + tet** | **LB** | **LB + tet** |
| **1** | 4.0 | 1.66 | 22.25 | 22.25 | 22.25 | 22.25 |
| **2** | 3.68 | 2.76 | 21.13 | 21.99 | 43.39 | 44.24 |
| **3** | 3.80 | 2.60 | 21.30 | 21.17 | 64.69 | 65.41 |
| **4** | 3.88 | 3.00 | 21.28 | 21.46 | 85.97 | 86.87 |

**Table 2. OD₆₀₀ values for each passage of E. coli cells with pDM/TRX+TIIIAlaMut plasmid after 22 hours of incubation and number of generations**

| **Passage number** | **OD₆₀₀** | | **Number of generations** | | | |
|---|---|---|---|---|---|---|
| | | | **per day** | | **total** | |
| | **LB** | **LB + tet** | **LB** | **LB + tet** | **LB** | **LB + tet** |
| **1** | 3.1 | 2.3 | 28.90 | 28.90 | 28.90 | 28.90 |
| **2** | 3.9 | 3.0 | 28.23 | 28.28 | 57.13 | 57.18 |
| **3** | 4.3 | 2.5 | 28.04 | 27.63 | 85.17 | 84.81 |
| **4** | 4.4 | 3.2 | 27.93 | 28.25 | 113.10 | 113.06 |

The results of colony counts and calculations of titers of *E*. *coli* cells with the pDM/TRX+TIIIA plasmid and with the pDM/TRX+TIIIAlaMut plasmid are shown in Table 3 and Table 4, respectively.

The total number of colonies per plate was calculated by multiplying the number of colonies counted on 1/8 plate by 8. Plasmid retention was calculated as the ratio of titers in the presence of the selection marker (tetracycline) to without it.

**Table 3. Calculations of titers of E. coli with pDM/TRX+TIIIA plasmid**

| **Passage** | **Number of colonies per plate** | | **Culture titer** | | |
|---|---|---|---|---|---|
| | **LB** | **LB + tet** | **LB [×10¹¹]** | **LB + tet [×10¹⁰]** | **Plasmid retention** |
| **1** | 17,808 | 6024 | 1.8 | 6.0 | 34% |
| **2** | 15,400 | 5256 | 1.5 | 5.3 | 34% |
| **3** | 13,936 | 5112 | 1.4 | 5.1 | 37% |
| **4** | 12,712 | 1992 | 1.3 | 2.0 | 16% |

**Table 4. Calculations of titers of E. coli with pDM/TRX+TIIIAlaMut plasmid**

| **Passage** | **Number of colonies per plate** | | **Culture titer** | | **Plasmid retention** |
|---|---|---|---|---|---|
| | **LB** | **LB + tet** | **LB** | **LB + tet** | |
| **1** | 1880 | 1480 | 1.88x10¹² | 1.48x10¹² | 78.7% |
| **2** | 3608 | 1336 | 3.61x10¹² | 1.34x10¹² | 37.1% |
| **3** | 4168 | 1720 | 4.17x10¹² | 1.72x10¹² | 41.2% |
| **4** | 5440 | 2240 | 5.44x10¹² | 2.24x10¹² | 41.2% |

The expression levels of TRX::TIIIA and TRX::TIIIAlaMut fusion proteins were verified after each passage. The SDS-PAGE result for the total TRX::TIIIA lysate is shown in Fig. 13, while the result of SDS-PAGE for the total TRX::TIIIAlaMut lysate is shown in Fig. 14.

Plasmid retention was verified directly on agarose gel for all samples after plasmid isolation. The results are shown in Figs. 15-16.

The analysis in question showed that in the absence of selection pressure, the *E. coli* strain with pDM/TRX+TIIIA plasmid is stable until the second passage. On the other hand, in the presence of an antibiotic, the plasmid persists in the strain for three passages, after the fourth passage its amount decreases significantly, but this does not significantly affect the amount of protein produced.

In the absence of selection pressure, the *E. coli* strain with the pDM/TRX+TIIIAlaMut plasmid is stable until the second passage. On the other hand, in the presence of an antibiotic, the plasmid persists in the strain for four passages. In addition, the pDM/TRX+TIIIAlaMut plasmid retention is higher than that of the pDM/TRX+TIIIA plasmid.

In view of the above, the pDM/TRX+TIIIA and pDM/TRX+TIIIAlaMut plasmids should be considered stable.

### Example 8

### In vitro determination of recombinant µ-conotoxin activity on oocytes by patch-clamp method

In this example of embodiment, the activity of µ-conotoxins according to the invention with amino acid sequences SEQ ID NO: 2 and SEQ ID NO: 5 was determined against known µ-conotoxins SIIIA and CnIIIC. In particular, it was tested whether the new recombinant conotoxins TIIIA and TIIIAlaMut have sodium current blocking properties similar to that of the commercially available conotoxin CnIIIC. A summary of the samples tested is shown in Table 5.

**Table 5. Summary of samples subjected to activity testing with molecular masses and effective concentrations**

| **Name of sample to be measured** | **Sample content** | **Molecular weight (Da)** | **Sample weight [mg]** | **Effective concentration [µM]** |
|---|---|---|---|---|
| Toxin A | TIIIA | 2433 | 0.15 | 0.7-1 |
| Toxin B | SIIIA | 2232 | 0.5 | no data |
| Toxin C | TIIIAlaMut | 2375 | 0.62 | no data |
| Toxin D | CnIIIC | 2376 | 0.1 | 0.5-1 |

The activity of the recombinant conotoxins was determined by the patch-clamp method on Nav1.4 ion channels expressed on *Xenopus* oocytes. The ion channel expression system on *Xenopus* oocytes is ideal for the electrophysiological characterization of voltage-dependent ion channels due to the relatively low background noise of endogenous channels and large oocyte cell size. The patch-clamp method is a standard in electrophysiology. Its concept is to perform a measurement on a very small piece of the cell membrane, known as a patch. For this purpose, an electrode is used, which is placed inside a thin glass pipette, the tip of which has a diameter of about 1 µm. The inside of the micropipette is filled with a solution with a composition similar to that of the extracellular fluid, since the measurement takes place on the outside of the cell. During the experiment, the tip of the micropipette is in direct contact with the membrane surface, creating a stable contact, both mechanically and electrically. Under these conditions, the measuring electrode records the currents flowing through the section of membrane restricted by the pipette tip. The number of ion channels present on such a piece is small enough that it is possible to distinguish currents that flow through individual channels.

### Methodology

In the first step, mRNA encoding Nav1.4 ion channels was introduced into oocytes by microinjection. Preparation of oocytes for microinjection: 5-15 mL of oocytes were collected from *Xenopus* ovaries, then divided into small clusters (about 10-20 oocytes). An enzymatic solution was then used to dissociate the oocytes (30-90 min at room temperature) until the follicles of some oocytes were ruptured. The cells were then washed with ND96 solution at least 5 times to remove residual enzyme. Under the microscope, healthy oocytes in stages V and VI (>0.8 mm in diameter) were selected. Microinjection was performed on the day of collection. Approximately 100 oocytes were used for testing each time. Selected oocytes were then transferred to ND96 solution and injected with aliquots of about 50 mL of cRNA solution (automatic oocyte injector). The cultures were then incubated at 18 °C for 2-7 days, depending on the desired level of ion channel expression. Thus prepared oocytes with Nav1.4 ion channels expressed on them were used to measure ion channel conductance.

Responses were measured at room temperature 1-6 days after cRNA injection and recorded at -70 mV using amplifier with software. Recombinant conotoxins SIIIA, TIIIA, TIIIAlaMut, and synthetic standard CnIIIC at 1 µM were used respectively to activate the Nav1.4 ion channel. Rapid and reproducible solution exchange (<300 ms) was achieved with a 50-µL funnel-shaped oocyte chamber combined with a rapid vertical flow of solution fed through a collector mounted directly above the oocyte. Pulses of agonist were applied for 2 s at 4-minute intervals. Voltage-current curves obtained on the same cells before and after administration of the selected compound to the extracellular environment were then compared.

### Results

The results of the analysis are shown in Figs. 17-18 and in Table 6.

**Table 6. Toxin concentration causing 50% inhibition (IC₅₀) and 99% inhibition (IC₉₉) of the sodium ion current generated by the human NaV1.4 channel.**

| | **[IC₅₀]** | **[IC₉₉]** |
|---|---|---|
| TIIIA | 9.7 µM | 0.97 mM |
| TIIIAlaMut | 3.8 µM | 0.38 mM |
| CnIIIC standard | 33.6 nM | 3.3 µM |

The studies performed indicate that the recombinant conotoxins TIIIA, SIIIA, TIIIAlaMut at a concentration of 1 µM reduce the amplitude of sodium currents from voltage-dependent sodium channels in oocytes. The results confirm the activity of all recombinant conotoxins (Figs. 17-18). The IC₅₀ values, or the concentrations required to block 50% of the Nav1.4 ion channel for the recombinant conotoxins TIIIA and TIIIAlaMut are at 1 µM. The level of ion channel blocking for the conotoxin TIIIAlaMut is approximately three times higher than for the conotoxin TIIIA, Fig. 18.

Sequence list

Sequence number (SEQ ID NO:) 1
Sequence type: nucleotide sequence
Length: 423
Description: Nucleotide sequence of the construct according to the invention in the TRX::TIIIA
variant
Sequence:
ATGCATCACCATCATCACCATTCTGGTTCTTCTGACAAAATCATCCACCTGACCGACGACT
CTTTCGACACCGACGTTCTGAAAGCTGACGGTGCTATCCTGGTTGACTTCTGGGCTGAATG
GTGCGGTCCGTGCAAAAAGATCGCTCCGATCCTGGACGAAATCGCTGACGAATACCAGG
GTAAACTGACCGTTGCTAAACTGAACATCGACCAGAACCCGGGTACCGCTCCGAAATACG
GTATCCGTGGTATCCCGACCCTGCTGCTGTTCAAAAACGGTGAAGTTGCTGCAACCAAAG
TTGGTGCACTGTCTAAAGGTCAGCTGAAAGAATTCCTGGACGCTAACCTGGCTATGCGTC
ATGGCTGCTGCAAAGGCCCGAAAGGCTGCAGCAGCCGTGAATGCCGTCCGCAGCATTGCT
GC
Sequence number (SEQ ID NO:) 2
Sequence type: amino acid sequence
Length: 141
Description: Amino acid sequence of the recombinant protein encoded by the construct according to the
invention in the TRX::TIIIA variant
Sequence:
MHHHHHHSGSSDKIIHLTDDSFDTDVLKADGAILVDFWAEWCGPCKKIAPILDEIADEYQGKL
TVAKLNIDQNPGTAPKYGIRGIPTLLLFKNGEVAATKVGALSKGQLKEFLDANLAMRHGCCK
GPKGCSSRECRPQHCC

Sequence number (SEQ ID NO:) 3
Sequence type: amino acid sequence
Length: 22
Description: Linear peptide identical to the conotoxin TIIIA sequence
Sequence: RHGCCKGPKGCSSRECRPQHCC

Sequence number (SEQ ID NO:) 4
Sequence type: nucleotide sequence
Length: 423
Description: Nucleotide sequence of the construct according to the invention in the TRX::TIIIAlaMut
variant

Sequence:ATGCATCACCATCATCACCATTCTGGTTCTTCTGACAAAATCATCCACCTGACCG
ACGACTCTTTCGACACCGACGTTCTGAAAGCTGACGGTGCTATCCTGGTTGACTTCTGGGC
TGAATGGTGCGGTCCGTGCAAAAAGATCGCTCCGATCCTGGACGAAATCGCTGACGAATA
CCAGGGTAAACTGACCGTTGCTAAACTGAACATCGACCAGAACCCGGGTACCGCTCCGA
AATACGGTATCCGTGGTATCCCGACCCTGCTGCTGTTCAAAAACGGTGAAGTTGCTGCAA
CCAAAGTTGGTGCACTGTCTAAAGGTCAGCTGAAAGAATTCCTGGACGCTAACCTGGCTA
TGCGTCATGGCTGCTGCAAAGGCCCGAAAGGCTGCAGCAGCCGTGctTGCCGTCCGCAGC
ATTGCTGC

Sequence number (SEQ ID NO:) 5
Sequence type: amino acid sequence
Length: 141
Description: Amino acid sequence of the recombinant protein encoded by the construct according to the
invention in the TRX::TIIIAlaMut variant
Sequence:MHHHHHHSGSSDKIIHLTDDSFDTDVLKADGAILVDFWAEWCGPCKKIAPILDEIAD
EYQGKLTVAKLNIDQNPGTAPKYGIRGIPTLLLFKNGEVAATKVGALSKGQLKEFLDANLAM
RHGCCKGPKGCSSRACRPQHCC

Sequence number (SEQ ID NO:) 6
Sequence type: nucleotide sequence
Length: 3869
Description: Expression vector referred to as pDMR is a derivative of the pBR322 vector, and contains
the constitutive promoter deoP1P2, the tetracycline resistance gene, the tRNA-Arg gene encoding the
UCU/CCU anticodon, and the sequence of the TRX::TIIIA fusion protein
Sequence:
TTCTCATGTTTGACAGCTTATCATCGATAAGCTTTAATGCGGTAGTTTATCACAGTTAAAT
TGCTAACGCAGTCAGGCACCGTGTATGAAATCTAACAATGCGCTCATCGTCATCCTCGGC
ACCGTCACCCTGGATGCTGTAGGCATAGGCTTGGTTATGCCGGTACTGCCGGGCCTCTTG
CGGGATATCGTCCATTCCGACAGCATCGCCAGTCACTATGGCGTGCTGCTAGCGCTATAT
GCGTTGATGCAATTTCTATGCGCACCCGTTCTCGGAGCACTGTCCGACCGCTTTGGCCGCC
GCCCAGTCCTGCTCGCTTCGCTACTTGGAGCCACTATCGACTACGCGATCATGGCGACCA
CACCCGTCCTGTGGATCCTCTACGCCGGACGCATCGTGGCCGGCATCACCGGCGCCACAG
GTGCGGTTGCTGGCGCCTATATCGCCGACATCACCGATGGGGAAGATCGGGCTCGCCACT
TCGGGCTCATGAGCGCTTGTTTCGGCGTGGGTATGGTGGCAGGCCCCGTGGCCGGGGGAC
TGTTGGGCGCCATCTCCTTGCATGCACCATTCCTTGCGGCGGCGGTGCTCAACGGCCTCAA
CCTACTACTGGGCTGCTTCCTAATGCAGGAGTCGCATAAGGGAGAGCGTCGACCGATGCC
CTTGAGAGCCTTCAACCCAGTCAGCTCCTTCCGGTGGGCGCGGGGCATGACTATCGTCGC

CGCACTTATGACTGTCTTCTTTATCATGCAACTCGTAGGACAGGTGCCGGCAGCGCTCTGG
GTCATTTTCGGCGAGGACCGCTTTCGCTGGAGCGCGACGATGATCGGCCTGTCGCTTGCG
GTATTCGGAATCTTGCACGCCCTCGCTCAAGCCTTCGTCACTGGTCCCGCCACCAAACGTT
TCGGCGAGAAGCAGGCCATTATCGCCGGCATGGCGGCCGACGCGCTGGGCTACGTCTTGC
TGGCGTTCGCGACGCGAGGCTGGATGGCCTTCCCCATTATGATTCTTCTCGCTTCCGGCGG
CATCGGGATGCCCGCGTTGCAGGCCATGCTGTCCAGGCAGGTAGATGACGACCATCAGG
GACAGCTTCAAGGATCGCTCGCGGCTCTTACCAGCCTAACTTCGATCATTGGACCGCTGA
TCGTCACGGCGATTTATGCCGCCTCGGCGAGCACATGGAACGGGTTGGCATGGATTGTAG
GCGCCGCCCTATACCTTGTCTGCCTCCCCGCGTTGCGTCGCGGTGCATGGAGCCGGGCCA
CCTCGACCTGAATGGAAGCCGGCGGCACCTCGCTAACGGATTCACCACTCCAAGAATTGG
AGCCAATCAATTCTTGCGGAGAACTGTGAATGCGCAAACCAACCCTTGGCAGAACATATC
CATCGCGTCCGCCATCTCCAGCAGCCGCACGCGGCGCATCTCGGGCAGCGTTGGGTCCTG
GCCACGGGTGCGCATGATCGTGCTCCTGTCGTTGAGTAATACGGTTATCCACAGAATCAG
GGGATAACGCAGGAAAGAACATGTGAGCAAAAGGCCAGCAAAAGGCCAGGAACCGTAA
AAAGGCCGCGTTGCTGGCGTTTTTCCATAGGCTCCGCCCCCCTGACGAGCATCACAAAAA
TCGACGCTCAAGTCAGAGGTGGCGAAACCCGACAGGACTATAAAGATACCAGGCGTTTC
CCCCTGGAAGCTCCCTCGTGCGCTCTCCTGTTCCGACCCTGCCGCTTACCGGATACCTGTC
CGCCTTTCTCCCTTCGGGAAGCGTGGCGCTTTCTCATAGCTCACGCTGTAGGTATCTCAGT
TCGGTGTAGGTCGTTCGCTCCAAGCTGGGCTGTGTGCACGAACCCCCCGTTCAGCCCGAC
CGCTGCGCCTTATCCGGTAACTATCGTCTTGAGTCCAACCCGGTAAGACACGACTTATCG
CCACTGGCAGCAGCCACTGGTAACAGGATTAGCAGAGCGAGGTATGTAGGCGGTGCTAC
AGAGTTCTTGAAGTGGTGGCCTAACTACGGCTACACTAGAAGGACAGTATTTGGTATCTG
CGCTCTGCTGAAGCCAGTTACCTTCGGAAAAAGAGTTGGTAGCTCTTGATCCGGCAAACA
AACCACCGCTGGTAGCGGTGGTTTTTTTGTTTGCAAGCAGCAGATTACGCGCAGAAAAAA
AGGATCTCAAGAAGATCCTTTGATCTTTTCTACGGGGTCTGACGCTCAGTGGAACGAAAA
CTCACGTTAACGATCGATTTTTGGTGATAATGTCCCAAATATGTCCCACTCTGAAATTATG
GAGGATATAAAGAAGGCGTAACTGATTGAATTGTAATGGCGCGCCCTGCAGGATTCGAA
CCTGCGGCCCACGACTTAGAAGGTCGTTGCTCTATCCAACTGAGCTAAGGGCGCGTTGAT
ACCGCAATGCGGTGTAATCGCGTGAATTATACGGTCAACCCTTGCTGAGTCAATGGCTTT
TGATCTGGGAGCTCGGGACCGTTGGCGATGTGCGGTTTGCTACATTCACAGATGTTCTTCG
CCACTTCCAGCAGCAGGTCATCAGGGGTGATTTCAGGATCGTAGATAAAGGTCAGGTTCG
GTGAAACCTGCTTCAACTCTGCATCTGCACGTAAGATCGCGCGGGTAATGGGCGAATCAG
ACGGGCCGATATTGGCGTGCATAAAGGCGTCTGGCAGGGTTCTGTCGAGGTAACGCCAG
AAACGTTTTATTCGAACATCGATCTCGTCTTGTGTTAGAATTAATTCTAACATACGGTTGC
AACAACGCATCCAGTTGCCCCAGGTAGACCGGCATCGATGTGACCGACGGTACGTGGTG
GTAAAGAATGGTCAGCAGAGAGAGTGCGTCATCAAGATCTTTCGCGCCTTCCAGCTCCAG
CCATTCGGAACCGTTCGCCAGAAAACGGGCGTAATCGGGTAAGACATAGCGCGGTTTGTA

CGGCGCATGACCTTCAAACATATCGCAGATTACACCTTCATCCAGCGCGCGGCGGGCTTC
GGCAGGAAGCTGTGGGTAAGGCAGATTGTTTTCTGCTTCCAGTGCCAGAAAATGGCGCTT
CTGCTCCGGGCTAAGCACTGGGCTGGTGACAATTTGCTGGCAACGTTGTTGCAGTGCATT
TTCATGAGAAGTGGGCATCTTCTTTTCCTTTTATGCCGAAGGTGATGCGCCATTGTAAGAA
GTTTCGTGATGTTCACTTTGATCCTGATGCGTTTGCCACCACTGACGCATTCATTTGAAAG
TGAATTATTTGAACCAGATCGCATTACAGTGATGCAAACTTGTAAGTAGATTTCCTTAATT
GTGATGTGTATCGAAGTGTGTTGCGGAGTAGATGTTAGAATACTAACAAACTCGCAAGGT
GAATTTTATTGGCGACAAGCCTAGGTTTGTTTAACTTTAAGGAGAAATCATATGCATCAC
CATCATCACCATTCTGGTTCTTCTGACAAAATCATCCACCTGACCGACGACTCTTTCGACA
CCGACGTTCTGAAAGCTGACGGTGCTATCCTGGTTGACTTCTGGGCTGAATGGTGCGGTC
CGTGCAAAAAGATCGCTCCGATCCTGGACGAAATCGCTGACGAATACCAGGGTAAACTG
ACCGTTGCTAAACTGAACATCGACCAGAACCCGGGTACCGCTCCGAAATACGGTATCCGT
GGTATCCCGACCCTGCTGCTGTTCAAAAACGGTGAAGTTGCTGCAACCAAAGTTGGTGCA
CTGTCTAAAGGTCAGCTGAAAGAATTCCTGGACGCTAACCTGGCTATGCGTCATGGCTGC
TGCAAAGGCCCGAAAGGCTGCAGCAGCCGTGAATGCCGTCCGCAGCATTGCTGCTAATCT
AGACTCGAGTTCGTCTTCAAGAA

Sequence number (SEQ ID NO:) 7
Sequence type: nucleotide sequence
Length: 4462
Description: Expression vector referred to as pDM is a derivative of the pBR322 vector, and contains the constitutive promoter deoP1P2, the tetracycline resistance gene, and the sequence of the TRX::TIIIA fusion protein
Sequence:
GGACCGTTGGCGATGTGCGGTTTGCTACATTCACAGATGTTCTTCGCCACTTCCAGCAGCA
GGTCATCAGGGGTGATTTCAGGATCGTAGATAAAGGTCAGGTTCGGTGAAACCTGCTTCA
ACTCTGCATCTGCACGTAAGATCGCGCGGGTAATGGGCGAATCAGACGGGCCGATATTGG
CGTGCATAAAGGCGTCTGGCAGGGTTCTGTCGAGGTAACGCCAGAAACGTTTTATTCGAA
CATCGATCTCGTCTTGTGTTAGAATTAATTCTAACATACGGTTGCAACAACGCATCCAGTT
GCCCCAGGTAGACCGGCATCGATGTGACCGACGGTACGTGGTGGTAAAGAATGGTCAGC
AGAGAGAGTGCGTCATCAAGATCTTTCGCGCCTTCCAGCTCCAGCCATTCGGAACCGTTC
GCCAGAAAACGGGCGTAATCGGGTAAGACATAGCGCGGTTTGTACGGCGCATGACCTTC
AAACATATCGCAGATTACACCTTCATCCAGCGCGCGGCGGGCTTCGGCAGGAAGCTGTGG
GTAAGGCAGATTGTTTTCTGCTTCCAGTGCCAGAAAATGGCGCTTCTGCTCCGGGCTAAG
CACTGGGCTGGTGACAATTTGCTGGCAACGTTGTTGCAGTGCATTTTCATGAGAAGTGGG
CATCTTCTTTTCCTTTTATGCCGAAGGTGATGCGCCATTGTAAGAAGTTTCGTGATGTTCA
CTTTGATCCTGATGCGTTTGCCACCACTGACGCATTCATTTGAAAGTGAATTATTTGAACC

AGATCGCATTACAGTGATGCAAACTTGTAAGTAGATTTCCTTAATTGTGATGTGTATCGA
AGTGTGTTGCGGAGTAGATGTTAGAATACTAACAAACTCGCAAGGTGAATTTTATTGGCG
ACAAGCCTAGGTTTGTTTAACTTTAAGGAGAAATCATATGCATCACCATCATCACCATTCT
GGTTCTTCTGACAAAATCATCCACCTGACCGACGACTCTTTCGACACCGACGTTCTGAAA
GCTGACGGTGCTATCCTGGTTGACTTCTGGGCTGAATGGTGCGGTCCGTGCAAAAAGATC
GCTCCGATCCTGGACGAAATCGCTGACGAATACCAGGGTAAACTGACCGTTGCTAAACTG
AACATCGACCAGAACCCGGGTACCGCTCCGAAATACGGTATCCGTGGTATCCCGACCCTG
CTGCTGTTCAAAAACGGTGAAGTTGCTGCAACCAAAGTTGGTGCACTGTCTAAAGGTCAG
CTGAAAGAATTCCTGGACGCTAACCTGGCTATGCGTCATGGCTGCTGCAAAGGCCCGAAA
GGCTGCAGCAGCCGTGAATGCCGTCCGCAGCATTGCTGCTAATCTAGAGTCGACCTGCAG
GCATGCAAGCTTAGCCCGCTTAATGAGCGGGCTTTTTTTTCTCGAGGACGTCTAAGAAAC
CATTATTATCATGACATTAACCTATAAAAATAGGCGTATCACGAGGCCCTTTCGTCTTCAA
GAATTCTCATGTTTGACAGCTTATCATCGATAAGCTTTAATGCGGTAGTTTATCACAGTTA
AATTGCTAACGCAGTCAGGCACCGTGTATGAAATCTAACAATGCGCTCATCGTCATCCTC
GGCACCGTCACCCTGGATGCTGTAGGCATAGGCTTGGTTATGCCGGTACTGCCGGGCCTC
TTGCGGGATATCGTCCATTCCGACAGCATCGCCAGTCACTATGGCGTGCTGCTAGCGCTA
TATGCGTTGATGCAATTTCTATGCGCACCCGTTCTCGGAGCACTGTCCGACCGCTTTGGCC
GCCGCCCAGTCCTGCTCGCTTCGCTACTTGGAGCCACTATCGACTACGCGATCATGGCGA
CCACACCCGTCCTGTGGATCCTCTACGCCGGACGCATCGTGGCCGGCATCACCGGCGCCA
CAGGTGCGGTTGCTGGCGCCTATATCGCCGACATCACCGATGGGGAAGATCGGGCTCGCC
ACTTCGGGCTCATGAGCGCTTGTTTCGGCGTGGGTATGGTGGCAGGCCCCGTGGCCGGGG
GACTGTTGGGCGCCATCTCCTTGCATGCACCATTCCTTGCGGCGGCGGTGCTCAACGGCCT
CAACCTACTACTGGGCTGCTTCCTAATGCAGGAGTCGCATAAGGGAGAGCGTCGACCGAT
GCCCTTGAGAGCCTTCAACCCAGTCAGCTCCTTCCGGTGGGCGCGGGGCATGACTATCGT
CGCCGCACTTATGACTGTCTTCTTTATCATGCAACTCGTAGGACAGGTGCCGGCAGCGCTC
TGGGTCATTTTCGGCGAGGACCGCTTTCGCTGGAGCGCGACGATGATCGGCCTGTCGCTT
GCGGTATTCGGAATCTTGCACGCCCTCGCTCAAGCCTTCGTCACTGGTCCCGCCACCAAA
CGTTTCGGCGAGAAGCAGGCCATTATCGCCGGCATGGCGGCCGACGCGCTGGGCTACGTC
TTGCTGGCGTTCGCGACGCGAGGCTGGATGGCCTTCCCCATTATGATTCTTCTCGCTTCCG
GCGGCATCGGGATGCCCGCGTTGCAGGCCATGCTGTCCAGGCAGGTAGATGACGACCATC
AGGGACAGCTTCAAGGATCGCTCGCGGCTCTTACCAGCCTAACTTCGATCATTGGACCGC
TGATCGTCACGGCGATTTATGCCGCCTCGGCGAGCACATGGAACGGGTTGGCATGGATTG
TAGGCGCCGCCCTATACCTTGTCTGCCTCCCCGCGTTGCGTCGCGGTGCATGGAGCCGGG
CCACCTCGACCTGAATGGAAGCCGGCGGCACCTCGCTAACGGATTCACCACTCCAAGAAT
TGGAGCCAATCAATTCTTGCGGAGAACTGTGAATGCGCAAACCAACCCTTGGCAGAACAT
ATCCATCGCGTCCGCCATCTCCAGCAGCCGCACGCGGCGCATCTCGGGCAGCGTTGGGTC
CTGGACTCACCAGTCACAGAAAAGCATCTTACGGATGGCATGACAGTAAGAGAATTATG

CAGTGCTGCCATAACCATGAGTGATAACACTGCGGCCAACTTACTTCTGACAACGATCGG
AGGACCGAAGGAGCTAACCGCTTTTTTGCACAACATGGGGGATCATGTAACTCGCCTTGA
TCGTTGGGAACCGGAGCTGAATGAAGCCATACCAAACGACGAGCGTGACACCACGATGC
CTGCAGCAATGGCAACAACGTTGCGCAAACTATTAACTGGCGAACTACTTACTCTAGCTT
CCCGGCAACAATTAATAGACTGGATGGAGGCGGATAAAGTTGCAGGACCACTTCTGCGCT
CGGCCCTTCCGGCTGGCTGGTTTATTGCTGATAAATCTGGAGCCGGTGAGCGTGGGTCTC
GCGGTATCATTGCAGCACTGGGGCCAGATGGTAAGCCCTCCCGTATCGTAGTTATCTACA
CGACGGGGAGTCAGGCAACTATGGATGAACGAAATAGACAGATCGCTGAGATAGGTGCC
TCACTGATTAAGCATTGGTAACTGTCAGACCAAGTTTACTCATATATACTTTAGATTGATT
TAAAACTTCATTTTTAATTTAAAAGGATCTAGGTGAAGATCCTTTTTGATAATCTCATGAC
CAAAATCCCTTAACGTGAGTTTTCGTTCCACTGAGCGTCAGACCCCGTAGAAAAGATCAA
AGGATCTTCTTGAGATCAAAGGATCTTCTTGAGATCCTTTTTTTCTGCGCGTAATCTGCTG
CTTGCAAACAAAAAAACCACCGCTACCAGCGGTGGTTTGTTTGCCGGATCAAGAGCTACC
AACTCTTTTTCCGAAGGTAACTGGCTTCAGCAGAGCGCAGATACCAAATACTGTCCTTCT
AGTGTAGCCGTAGTTAGGCCACCACTTCAAGAACTCTGTAGCACCGCCTACATACCTCGC
TCTGCTAATCCTGTTACCAGTGGCTGCTGCCAGTGGCGATAAGTCGTGTCTTACCGGGTTG
GACTCAAGACGATAGTTACCGGATAAGGCGCAGCGGTCGGGCTGAACGGGGGGTTCGTG
CACACAGCCCAGCTTGGAGCGAACGACCTACACCGAACTGAGATACCTACAGCGTGAGC
TATGAGAAAGCGCCACGCTTCCCGAAGGGAGAAAGGCGGACAGGTATCCGGTAAGCGGC
AGGGTCGGAACAGGAGAGCGCACGAGGGAGCTTCCAGGGGGAAACGCCTGGTATCTTTA
TAGTCCTGTCGGGTTTCGCCACCTCTGACTTGAGCGTCGATTTTTGTGATGCTCGTCAGGG
GGGCGGAGCCTATGGAAAAACGCCAGCAACGCGGCCTTTTTACGGTTCCTGGCCTTTTGC
TGGCCTTTTGCTCACATGTTCTTTCCTGCGTTATCCCCTGATTCTGTGGATAACCGTATTAC
CGCCTTTGAGTGAGCTGATACCGCTCGCCGCAGCCGAACGACCGAGCGCAGCGAGTCAGT
GAGGATCGATCCG

## Claims

1. A construct for an expression vector for recombinant µ-conotoxin TIIIA or TIIIAlaMut expression, **characterized in that** it contains either the nucleotide sequence of µ-conotoxin TIIIA SEQ ID NO: 1 or the nucleotide sequence of µ-conotoxin TIIIAlaMut SEQ ID NO: 4, both of which at the 5' end contain a sequence encoding six histidines (6His) linked by a serine-glycine-serine linker (SGS) to a construct encoding either TRX::TIIIA fusion protein containing the µ-conotoxin *TIIIA* gene and the gene encoding a leader protein, or a TRX::TIIIAlaMut fusion protein containing the µ-conotoxin *TIIIAlaMut* gene and a leader protein, where the said leader protein is thioredoxin (TRX) modified by point mutagenesis in which the amino acid methionine at position 37 has been replaced by lysine.

2. An expression vector containing the construct as defined in claim 1 under the control of a constitutive promoter.

3. The vector according to claim 2, **characterized in that** the constitutive promoter is *deoP1P2.*

4. The vector according to claim 2 or 3, **characterized in that** it is an expressing vector pDM, preferably pDM with the nucleotide sequence SEQ ID NO: 7, or pDMR, preferably pDMR with the nucleotide sequence SEQ ID NO: 6.

5. An isolated *E. coli* cell containing the expression vector as defined in any of the preceding claims 2 to 4.

6. The cell according to claim 5, **characterized in that** it is an *E. coli* S4B cell.

7. A method for producing µ-conotoxin TIIIA or TIIIAlaMut in *E. coli* with the expression vector containing the construct as defined in claim 1, **characterized in that** it includes the following steps:
a) Transformation of an *E. coli* cell with the expression vector containing the construct according to claim 1, under the control of a constitutive promoter, encoding either TRX::TIIIA fusion protein with amino acid sequence SEQ ID NO: 2 or a TRX::TIIIAlaMut fusion protein with SEQ ID NO: 5;
b) Expression of the TRX::TIIIA or TRX::TIIIAlaMut fusion protein;
c) Isolation and purification of the TRX::TIIIA or TRX::TIIIAlaMut fusion protein;
d) Disulfide bridges forming by subjecting the purified TRX::TIIIA or TRX::TIIIAlaMut fusion protein to glutathione treatment in GSH/GSSG and dialysis in buffer;
e) Cyanogen bromide cleavage of the TRX::TIIIA or TRX::TIIIAlaMut fusion protein with disulfide bridges formed;
f) Purification of the cleaved TIIIA or TIIIAlaMut peptide.

8. The method according to claim 7, **characterized in that** the constitutive promoter used in step a) is *deoP1P2.*

9. The method according to claims 7 or 8, **characterized in that** the expression vector used in step a) is pDM, preferably pDM with the nucleotide sequence SEQ ID NO: 7, or pDMR, preferably pDMR with the nucleotide sequence SEQ ID NO: 6.

10. The method according to any of the preceding claims 7 to 9, **characterized in that** the transformed cell is the *E. coli* S4B cell.

11. The method according to any of the preceding claims 7 to 10, **characterized in that** in step a) the transformation is carried out by electroporation.

12. The method according to any of the preceding claims 7 to 11, **characterized in that** in step b) the culture of the transformed expressing *E*. *coli* strain is conducted at 30 °C.

13. The method according to any of the preceding claims 7 to 12, **characterized in that** in step e) the cleavage is carried out using cyanogen bromide given in a molar excess of 100:1 relative to the methionine residues.

14. The method according to any of the preceding claims 7 to 13, **characterized in that** in step e) the weight ratio of cyanogen bromide to the cleaved fusion protein is 1.375:1.

15. The method according to any of the preceding claims 7 to 14, **characterized in that** in step e) the cleavage is carried out in 0.1M HCl for 3 hours at room temperature, in the dark with stirring.
